Europäisches Patentamt

European Patent Office

Office européen des brevets

(11) Publication number: **0 085 277**
**B1**

(12) **EUROPEAN PATENT SPECIFICATION**

(45) Date of publication of patent specification: **11.09.85**

(21) Application number: **82810543.7**

(22) Date of filing: **15.12.82**

(51) Int. Cl.⁴: **C 07 C 99/10,** C 07 C 101/12, C 07 C 121/43

(54) Process for the production of ethylenediamine tetraacetic acid.

(30) Priority: **21.12.81 US 333138**

(43) Date of publication of application: **10.08.83 Bulletin 83/32**

(45) Publication of the grant of the patent: **11.09.85 Bulletin 85/37**

(84) Designated Contracting States: **BE CH DE FR GB IT LI**

(56) References cited:
**DE-B-1 064 952**
**GB-A-1 159 326**

(73) Proprietor: **CIBA-GEIGY AG Postfach CH-4002 Basel (CH)**

(72) Inventor: **Singer, John Jacob 143 Worcester Road Hollis New Hampshire 03049 (US)**

Courier Press, Leamington Spa, England.

## Description

This invention relates to a process for the production of ethylene-diamine tetraacetic acid (EDTA) where the process is based on a two-stage cyanomethylation of the ethylenediamine.

In US 2,855,428 various processes for the preparation of aminoacid-type chelating, metal ion sequestering agents are described. Among these general processes is the coupling of the amine with chloracetic acid as disclosed in US 2,130,505. Another method is based on the oxidation of an amine with an ethanol group on the amine nitrogen as exemplified in US 2,384,816. A third more commonly used method is via the appropriate nitrile, as originally mentioned in US 2,407,645. Variations from this direct hydrolysis of the nitrile as formed, include the separate formation, separation and hydrolysis of the nitrile as described in US 2,164,781 and US 2,205,995.

The aforementioned US 2,855,428 is also related to the direct synthesis of the nitrile. The nitrile is therein synthesized by introducing the amine or ammonia into an acidic medium containing the formaldehyde and the hydrocyanic acid. The nitrile as formed is directly precipitated in the acidic medium and is recovered therefrom in good yields.

US 3,424,783 refers to a variant of US 2,855,428 where the cyanomethylation is conducted in acidic media. US 3,644,444, US 3,758,534, US 3,679,729 and US 3,714,223 are similar variants disclosing varying specific acidic pH ranges and temperature ranges, but in each case the nitrile is always isolated and washed before saponificature as a prerequisite to pure ethylenediamine tetraacetic acid preparation. Failure to treat the reaction product as described therein results in lower yield, or requires more sophisticated equipment and extensive waste treatment operations.

Several of the above mentioned references including US 3,959,342 discuss the nitrile route via nitrilotriacetonitrile (NTN) and its hydrolysis acid to the corresponding (NTA). The cyanomethylation to NTN is always performed under acidic conditions. In general, these procedures provide yields of NTN in the range 80 to about 95 wt% (based on ammonia or hexamethylene tetramine). The resulting products are eminently suitable sequestering agents in laundry compositions.

It is an object of this invention to provide EDTA in substantially pure form and in almost quantitative yields using techniques which permit the use of simplified equipment and which minimize hazard and enviromental contamination from the hydrocyanic acid used and the by-products normally produced by older processes. In addition, output per unit volume of reactor is increased and the yield, based on amine used, is essential quantitative. From this pure EDTA, EDTA salts are obtained in excellent purity and essentially quantitative yield.

More particularly, it is one object of this invention to provide a process for the production of pure ethylenediamine tetraacetic acid which comprises the steps of

(a) admixing 2 moles of formaldehyde with 1 mole of ethylenediamine to form an adduct thereof,

(b) performing a first stage cyanomethylation of said adduct by reacting it with 2 moles of hydrocyanic acid at a temperature below about 30°C to form ethylenediamine diacetonitrile,

(c) adding at least 2 additional moles each of hydrocyanic acid and formaldehyde to said ethylenediamine diacetonitrile in a second cyanomethylation step in the presence of sufficient acid to maintain the pH-value of the reaction mixture below about 1.0 and temperatures below about 70°C to complete the second stage of cyanomethylation and to form ethylenediamine tetraacetonitrile, and

(d) saponifying the ethylenediamine tetraacetonitrile with a strong caustic solution to form the tetrasodium salt solution of ethylene-diamine tetraacetic acid.

Another object of the invention is to provide a process for the production of ethylenediamine tetraacetonitrile (EDTN).

(EDTA) and its salts can be prepared in high quality and almost quantitative yield by varying the process according to US 2,855,428 whereby (a) an adduct is formed from an admixture of two moles of formaldehyde with one mole of ethylenediamine. This adduct is then subjected to (b) a first stage of cyanomethylation by the addition of two moles of hydrocyanic acid. This acid is added while maintaining the reaction mixture at a pH-value of about 8—10 and — optionally by cooling — at a temperature below about 30°C to form ethylenediamine diacetronitrile (EDDiN). The EDDiN is soluble and remains dissolved in the reaction mixture.

The EDDiN is then subjected to (c) a second stage of cyanomethylation by the introduction of the EDDiN solution into an acidic mixture at a pH-value of about below 2, preferably about 1, and more preferably 0.5. The acidic solution contains a strong, preferably mineral acid such as sulphuric acid keeping the solution at the desired pH-value, two moles each of hydrocyanic acid and formaldehyde. A slight (10 mole %) excess of hydrocyanic acid is preferred to assure high yields. The pH-value during the introduction of the EDDiN is preferably maintained below about 1.0 by acid and by controlling the rate of EDDiN addition. The exotherm of this second stage cyanomethylation is readily controlled by pre-cooling the acidic reaction medium so that the temperature during this second stage cyanomethylation preferably stays below about 70°C until the reaction is completed and all the EDTN is precipitated. The heat released in the reaction is absorbed in the sensible heat rise of the reaction medium or mass to 70°C. No directly applied external cooling need to be used.

A portion of the precipitated EDTN, slurried in the aqueous mother liquor, is separated. The mother liquid is cooled, preferably to 20 to 30°C, and reintroduced into the second stage cyanomethylation step as an adjunct coolant for maintaining the reaction medium below about 70°C.

The EDTN may be washed and reslurried in water for hydrolysis to form EDTA and its salts. The EDTN slurry may be hydrolyzed in an alkaline medium by reacting with e.g. caustic soda to form a solution of the tetrasodium salt of EDTA (EDTA·Na$_4$). The ammonia released in this hydrolysis is distilled from the solution. The free acid, EDTA, is precipitated from the EDTA°Na$_4$ solution by adding sufficient acid to reduce the pH-value to below about 3, preferably to 2 to 3.

If any part of the EDTN synthesized by the above process is converted into EDTA by an acid precipitation, then the process described above can be further improved so as to eliminate the need for any hydrocyanic acid waste treatment and for any "HCN rated" equipment other than the primary reaction vessels which this acid is introduced as a reactant.

This second part of the invention takes advantage of the fact that EDTN settles from a slurry very rapidly and occupies a volume approximately 35% of the original slurry. After the formation of the EDTN is essentially complete the agitator in the reactor is stopped. After settling of the slurry, a portion of the supernatant liquid is removed by a syphon, "J" leg or through a properly positioned side opening for recycle as coolant. A "clean" separation of the mother liquor is not necessary since any nitrile removed with the mother liquor will be recycled into the next batch without loss.

Sufficient water is added to the EDTN thick slurry to permit it to be pumped. The agitated re-thinned slurry (approx. 30% solids) is pumped into a tank containing sufficient dilute caustic soda to neutralize the acid catalyst, preferably a strong acid such as e.g. sulphuric acid and to neutralize any remaining hydrocyanic acid to its sodium salt.

After neturalization is complete the pH-value should be in the range of from 9 to 11. This is not critical except the pH-value should be high enough so that colour will not form by the decomposition and hydrolysis of sodium cyanide. Because the slurry has been cooled by the addition of cold water, no saponification of the EDTN will occur even if fairly substantial amounts of free caustic soda are present.

At this point the alkaline slurry can be separated by a common filter or centrifuge since the vapor pressure of the hydrocyanic acid in the filtrate is very low — substantially less than its vapor pressures over the cyanide electro-plating solutions commonly used in the metal working industry. Safe working conditions can be maintained with standard ventilating equipment. After centrifuging and washing, the pure EDTN may be reslurried with water and saponified in the conventional manner.

The alkaline centrifugate and wash water is temporarily stored in a holding tank until the next batch of EDTN is converted into EDTA salts.

If the manufacturing decision has been made to convert the EDTN to EDTA then the acidified nitrile slurry described above (the initial reaction product) is pumped directly to the saponification vessel with no attempt being made to separate the acid catalyst. Any alkaline supernatant liquor or wash water obtained when the pure EDTN was isolated, is now also pumped into the saponification vessel which contains sufficient caustic soda to permit complete neutralization and saponification. Excess caustic is permissible. During this saponification reaction, any unreacted sodium cyanide may be treated with additional formaldehyde to carry on a "Bersworth" type synthesis which eliminates the excess of sodium cyanide.

The solution resulting from this modified saponification reaction is now acidified to a pH-value of about 3, or more preferably about 2, with mineral acid such as sulphuric acid e.g., and EDTA is precipitated separated from the resulting slurry and washed with water until free from salts and mineral acid. The quality and percent recovery of EDTA from this process is comparable in quality and recovery to EDTA prepared from purified nitrile. The acid mother liquor may then be neutralized to destroy the excess acid catalyst (sulphuric acid, e.g.). This solution contains only the sodium salt of the acid catalyst (sodium sulfate, e.g.) and harmless sodium salts of organic acids which are biodegradable. The cumbersome, costly and potentially hazardous treatment of cyanide waste liquor is unnecessary when the above process is used.

The following reaction equations in Table 1 summarise the process chemistry:

**0 085 277**

TABLE 1

(a)  Adduct Formation:

$$H_2N-CH_2CH_2-NH_2 \ + \ 2CH_2O \ \xrightarrow[\text{pH} \sim 10]{<30\,°C} \ \text{Adduct}$$

| EDA | Formaldehyde | |
|-----|-----|-----|
| MW 60 | MW 30 | MW 120 |

(b)  First Stage Cyanomethylation (EDDiN–Formation):

$$\text{Adduct} \ + \ 2HCN \ \xrightarrow[\text{pH} \sim 10]{<30\,°C} \ NC-CH_2-NH-CH_2CH_2-NH-CH_2-CN$$

$$-2H_2O$$

EDDiN

MW 138

(c)  Second Stage Cyanomethylation (EDTN–Formation):

$$EDDiN \ + \ 2HCN \ + \ 2CH_2O \ \xrightarrow[\text{pH} \ 1]{<70°C} \ (NC-CH_2)_2N-CH_2CH_2-N(CH_2-CN)_2$$

$$-2H_2O$$

EDTN

MW 216

(d) Hydrolysis of EDTN:

$$EDTN \ + \ 4NaOH \ \xrightarrow[-4NH_3]{H_2O} \ (NaOOC-CH_2)_2N-CH_2CH_2-N(CH_2-COONa)_2$$

$EDTA.Na_4$

MW 292 (as EDTA)

Table 2

| Reaction | Heat of Reaction | T Adiabatic |
|-----|-----|-----|
| (a) | −130.2 KJ/mole EDA | 150—200°C |
| (b) | −64.0 KJ/mole EDA | 60—75°C |
| (c) | −205.9 KJ/mole EDA | 30—35°C |

4

As the first stage cyanomethylation (b) must be controlled to below 30°C to prevent colour formation, it is also advantageous to also cool the adduct formation reaction (a) to below 30°C to provide a cooled adduct solution for the cyanomethylation reaction (b). This reduces the refrigeration required to prevent colour-forming side reactions therein which start at above about 30°C. This resulting cooled solution from this first stage cyanomethylation is kept below about 30°C when fed into the subsequent second stage cyanomethylation.

The second stage cyanomethylation reaction (c) is carried out using e.g. cooled (below 30°C) sulphuric acid to maintain the pH-value below about 1.0. Owing to the precipitation of EDTN during this reaction (c), it is difficult to cool the reaction mix during reaction in order to control the exotherm without fouling of heat transfer surfaces. The reaction is primarily carried out adiabatically using the sensible heat rise from below 30°C to 70°C by auxiliary cooling, supplied by adding recycled cooled mother liquor (after EDTN separation) to the reaction medium as required. The final temperature should be maintained below about 70°C to prevent side reactions and preferably about 65°C to ensure substantially quantitative completion of the EDTN formation. Recycled cooled mother liquor also may be initially charged as the cooled reaction medium.

The general synthesis scheme for EDTA according to this invention can be carried out in a batch process, in a semibatch process or in a continuous process. The best yields and quality have been obtained by the semi-batch procedure which is preferred. However, it is possible by careful monitoring and equilibrating of the conditions and feed-rates to realize good yields at low costs in a continuous flow process scheme.

The process will be most particularly discussed and detailed with respect to the preferred semi-batch processing.

The semi-batch process is carried out in two cyanomethylation stages wherein the first stage a previously prepared adduct of ethylene-diamine (EDA) with two moles formaldehyde is reacted with a stoichio-metric amount of hydrocyanic acid sufficient to form the dinitrile, EDDiN. The reaction mass remains in solution. Temperature control of the solutions is readily carried out by removal of the considerable heats of the reactions from both the formation of the adduct and the formation of the dinitrile. The temperature of this first stage is controlled to below about 30°C. The reaction is substantially complete within about half an hour.

The second stage cyanomethylation produces crystalline EDTN by reacting the EDDiN with slight excesses of formaldehyde and hydrocyanic acid at a pH-value of below 1.0. The quality and yield of EDTN in this second stage are not adversely affected by reaction temperatures below about 70°C, and so the reaction is preferably carried out adiabatically, without external heat exchange, but utilizing the rise in temperature of the reaction mass from below 30°C to below 70°C. This obviates deposition of solids onto the cooling surfaces of heat exchangers. The temperature of the slurry before and during the reaction is controlled within the prescribed range by recycling cooled mother liquor from previous batches in required amounts to the reaction mass before and during the reaction.

Upon completion of the second stage cyanomethylation, EDTN, which forms rapidly as large crystals, may be separated from the mother liquor, washed, preferably with water, and then hydrolyzed. The hydrolysis takes place in a caustic medium to produce very pure EDTA°Na$_4$ in solution. This is a stock solution for the formation of further EDTA products such as the acid and various salts.

Alternatively, the nitrile may be saponified without separation from all or part of the mother liquor or reaction liquor and then (if necessary to remove residual hydrocyanic acid after treatment with formaldehyde, precipitated with acid at a pH-value 3 or lower to form EDTA in essentially quantitative yield of high purity.

Ammonia, which is liberated by the hydrolysis of the nitrile groups is recovered, preferably in anhydrous form as a useful by-product. The blowdown from the EDTN formation and separation contains the excess formaldehyde and hydrocyanic acid from the EDTN separation step. This is processed by heating to about 80°C under alkaline conditions to destroy the acid before the liquid is discharged into an effluent system.

Specifically as regards each step it must be kept in mind that the EDDiN formation is sensitive to temperature and the reaction and subsequent handling of the solution thereof be kept below about 30°C. If the temperature exceeds 30°C colour formation is significant and a reduction in yield and purity results.

The process steps (a) to (d) are now explained in detail:

(a) Adduct Formation

To facilitate the heat removal, the adduct formation step is preferably separated from the first stage cyanomethylation. As seen in Table 2, the mixture of the two moles of formaldehyde with the EDA to form the adduct liberates twice the heat as does the formation of the dinitrile (i.e. −130.2 KJ vs. −63.2 KJ). Any cooling system of sufficient capacity can be used to remove this significant quantity of heat; such as cooled holding tanks in which the aqueous solution of formaldehyde (37—50%) is reacted with the EDA in 2:1 molar proportion. The pH-value of the adduct solution is about 9—10. Feed rates of these two components and cooling rates are adjusted in response to thermometric sensors. Preferably the final adduct temperature should be about 20—25°C. Care should be taken not to exceed 30°C during formation of the EDA/formaldehyde adduct.

5

(b) EDDiN Formation

The first stage dicyanomethylation is completed by reacting the EDA/formaldehyde adduct in aqueous solution with a stoichiometric quantity (2 moles) of hydrocyanic acid. This step of the reaction is most thermosensitive and is conducted below 30°C by feeding 100% hydrocyanic acid slowly below the surface of the adduct solution in a cooled reaction vessel. The exothermic reaction (Table 2) of the hydrocyanic acid with the adduct is moderately rapid. The reaction is substantially quantitative, i.e. more than 99%, within about 30 minutes after completion of the hydrocyanic acid addition. The solution of the EDDiN is then maintained at about 20—25°C and transferred to the next step.

(c) EDTN Formation

Solid EDTN is prepared by adding, at a controlled rate, the EDDiN solution to a mixture of sulphuric acid (catalyst), formaldehyde and hydrocyanic acid and recycled mother liquor (as coolant). Sufficient sulphuric acid is desirable to maintain the pH-value below about 1.0. Too rapid addition of the EDDiN solution causes the pH-value to rise with the promotion of the formation of side products and consequent reduction in yield. The pH-value of the reaction mass is monitored by pH-sensors and the rate of EDDiN addition is controlled and adjusted to maintain the suitably acidic medium below a pH-value of 1.0. A definite effect of addition rate vs. pH-value was noted and thus an upper desirable pH-limit of 1.0 was established.

It was also noted that an excess of hydrocyanic acid and formaldehyde promoted the second stage cyanomethylation. An almost quantitative conversion to EDTN was obtained at about 10% combined molar excess. (Table 3).

Table 3

| Hydrocyanic acid/ formaldehyde: EDDiN | % Molar Excess of hydrocyanic acid/ formaldehyde | EDTN Yield (% Theory) |
|---|---|---|
| 1.8/1.8:1 | (−5%) | 85.2 |
| 2.0/2.0:1 | 0% | 94.2 |
| 2.2/2.2:1 | 5% | 96 |
| 2.4/2.4:1 | 10% | 97.99 |

The product of the second stage cyanomethylation, i.e. EDDiN to EDTN, is more thermostable than that of the first stage cyanomethylation. The reaction can proceed with minimal undesired side reactions if the reaction mass temperature is kept below about 70°C and preferably at about 60—65°C to ensure substantial completion of the reaction within a reasonable time, i.e. one hour, after completion of EDDiN addition.

This reaction is exothermic, −205.1 KJ/mole using a 50% solution of formaldehyde, but it has been determined that the quality and yield of EDTN is not adversely affected by reaction temperatures below about 70°C. Colour (undesirably) develops above 70°C. The reaction is best carried out adiabatically in order to prevent fouling of heat exchange surfaces by formation of coatings of solids therein.

For control of temperature to below 70°C, and preferably to about 60—65°C, the initial mass is cooled by using cooled mother liquor from previous batches as a diluent of the reaction mass. Sufficient mother liquor recycled from the subsequent separation step is cooled and is then introduced into the reaction mass. The mother liquor is cooled to 20—30°C prior to recycling. Generally, the amount of coolant mother liquor required will maintain a slurry concentration in the reaction mass in the range of 17 to 23% EDTN solids. The addition of the cooled mother liquor is controlled by temperature sensors in the reaction mass.

Another advantage for using the recycled mother liquor for diluting and cooling the reaction mass is that the excess of hydrocyanic acid/formaldehyde used in this stage is reutilized by this recycling thus, reducing the overall consumption of these materials and also reducing the amounts of these materials which may have to be treated before release as a proper effluent.

A suitable treatment of the hydrocyanic acid/formaldehyde excesses before disposal is to strongly alkalize and then to heat to about 80°C. The hydrocyanic acid is decomposed to less than 100 ppm and the resulting ammonia is recovered. Approximately 60—70% of the filtrate is recycled.

(C1) EDTN Isolation

The EDTN reaction mass need not be cooled before the slurry is allowed to settle and a portion of the mother liquor removed for recycle. Any mother liquor removed must be cooled before reuse since it is a "heat sink" for the next reaction to be run. Cooling however, is simplified since the liquid contains only small amounts of nitrile as suspended particles, and conventional heat exchangers may be used with minimal fouling.

The hot concentrated slurry is adjusted to approximately 30% solids by the addition of cold water. After mixing for a few minutes the slurry is transferred to a holding tank containing enough sodium hydroxide to raise the pH-value of the slurry to 9.0 or higher when neutralization of the acids (hydrocyanic acid and sulphuric acid) is complete.

After acid neutralization the nitrile may be isolated using conventional centrifuges or filtering equipment. Ordinary ventilation equipment is adequate for worker safety. After isolation the nitrile may be reslurried with water and saponified in the conventional manner, the mother liquor and wash water being temporarily stored in holding tanks. If EDTA acid is to be prepared, the concentrate slurry is thinned with water after the recycle liquor has been removed. The thinned (approx. 30%) slurry is then pumped directly to the saponification tank along with any alkaline mother liquor and wash water from previous runs in which the nitrile was isolated and washed so that pure EDTA·Na$_4$ solution could be prepared.

### (d) Hydrolysis of EDTN

The stock product, aqueous solution of EDTA·Na$_4$ 38% (30% as EDTA), is produced by hydrolyzing the EDTN is caustic media. The aqueous slurry from the EDTN isolation step is introduced into an aqueous 33% sodium hydroxide solution at 100—105°C. At this concentration and temperature, the hydrolysis reaction is rapid and proceeds to completion with the evolution of 4 moles ammonia. While the reaction is exothermic, a net heat input is required to maintain reflux and facilitate the removal of the ammonia. An excess of sodium hydroxide is required to prevent excess colour formation in the bulk of the liquid stock solution.

A product of high quality is produced by heating the solution until ammonia ceases to evolve, if the nitrile had been previously separated and washed. If the acid nitrile slurry is used additional caustic must be added to guarantee the excess of sodium hydroxide. At this point the product concentration is approximately 30 wt % as EDTA.

Residual hydrocyanic acid is reduced to an acceptable level by treatment with small amounts of formaldehyde and heating to 80°C. Finally any incidental colour is bleached by treatment with a 35% solution of hydrogenperoxide. The assay of the stock solution product is adjusted to 30% as EDTA.

The alkaline treatments of the hydrocyanic acid and other various nitrile side products with formaldehyde forms soluble sodium glycolate and other sodium organic acid salts, all of which are biodegradable.

The semi-batch process, which is capable of automation and computer control is preferred but within the ambit of the invention, where the adduct [step (a)] is formed which is subsequently subjected to cyanomethylation to EDDiN at a pH-value of about 10 and then is cyanomethylated to EDTN at a pH-value below about 1.0, the process can also be continuously performed in continuous flow apparatus.

The previously formed and cooled adduct (1:2 moles) is fed into a continuously stirred vessel where it is reacted with a stream of hydrocyanic acid. The yields of EDDiN from this continuous first stage cyanomethylation (as determined by a batch-wise second stage cyanomethylation to EDTN) were about 50% EDDiN. The reactor temperature was kept below 30°C and the residence time in the reactor was about 30 minutes. Adjustments and variations in hydrocyanic acid concentration in the vessel indicate that, in such a continuous reactor, an initial deficiency of the hydrocyanic acid is not compensated nor does an excess ensure fuller completion of the reaction within the stated residence-time parameter.

EDDiN reacted in a continuous reactor train with additional hydrocyanic acid and formaldehyde in an acid medium showed yields of 75 to 96% of theoretical, based upon 100% EDDiN feed, depending upon dwell time and excess hydrocyanic acid and formaldehyde feeds. The higher yields were obtained with hydrocyanic acid and formaldehyde excesses ranging around 30 mole % at dwell times of from one to two hours. However, incremental yields, determined at fixed periods during extended runs (2—1/2 to 9 hours) are progressively lower at succeeding intervals (after establishing the reaction equilibrium) indicating accumulation of solid EDTN in the reactor train. Because of such solid accumulation in the continuous processing scheme, the semi-batch process is preferred. However, continuous processing according to the process of this invention has been performed.

Table 4 shows the flow diagram of the general reaction scheme including the steps and material feed for preparing EDTA via EDTN through the steps of (a) adduct formation, and (b) the EDDiN forming first-stage cyanomethylation to (c) EDTN slurry preparation after the second stage cyanomethylation to the optional subsequent (c 1) separation of EDTN and (d) its hydrolysis to form the EDTA·Na$_4$ bulk stock solution.

The directions for practicing the process of this invention set forth in the appended examples showing the preferred and alternate modes. It is understood that while specific apparatus is described in some of the examples, equivalent apparatus suitable for performing the described unit operations may be substituted.

## TABLE 4

## PROCESS FLOW DIAGRAM FOR EDTA-FORMATION

Example 1: (Semi-batch procedure)

To a 500 ml reactor flask equipped with a mechanical stirrer, Friedrichs condenser chilled with ice water, thermometer, 250 ml dropping funnel with dip tube, and an ice bath, charge 120.0 g of aqueous 50% formaldehyde. Charge to the dropping funnel 120.0 g of 100% EDA. Begin adding the EDA dropwise into the reactor at a rate so that the temperature is maintained below 30°C with the ice bath.

After the addition is complete, the adduct is formed. Slowly charge 54.0 g of 100% hydrocyanic acid through the dropping funnel and its dip tube maintained below the surface of the liquid adduct, so that the temperature is continuously maintained below 30°C by the ice bath.

After the addition is complete, stir for about 30 minutes. The EDDiN thus formed in solution is then ready for the second stage cyanomethylation to prepare EDTN.

To a 2-liter reactor flask, equipped with a mechanical stirrer, Friedrichs condenser chilled with ice water, thermometer, 250 ml dropping funnel, and pH-probe, charge 713 g of distilled water, 54.8 g of 96% sulphuric acid, and 140.0 g of 50% formaldehyde. Cool the reactor contents to 20°C with an ice bath and charge 64.8 g of 100% hydrocyanic acid, charge the EDDiN solution to the contents of the flask while following the pH-value of the reaction mass. Control and adjust the rate of addition so that the pH-value is maintained between 0.5 and 1.0 during the course of the addition. The reaction is carried out adiabatically. The temperature will rise from about 25—30°C to 60—65°C and is maintained at this upper level for about one half to one hour before isolation of the precipitated EDTN.

Cool the resulting slurry to 30°C, filter, and wash the wet cake with distilled water. Dry the wet cake under vacuum at 50°C.

Slurry the separated EDTN solid with sufficient 25%—33% sodium hydroxide to give a mole ratio of 1.0/4.4 EDTA to hydroxide. Pump the slurry from the slurry vessel to a second vessel containing a heel of hot (100—104°C) sodium EDTA solution (38%).

After the addition is complete, the reaction mass is held at reflux until no ammonia can be detected at the vent. The solution is then treated for trace cyanide by addition of hydrogen peroxide. Add water to dilute the solution to 30% EDTA content as EDTA·Na$_4$. Yield 98% of theory.

Example 2: (Continuous procedure)

The continuous preparation of EDTN is performed in two phases. The first phase consists of an evaluation of the first stage cyanomethylation reaction wherein the adduct is cyanomethylated in a continuously stirred reactor. The yield of reaction product (EDDiN) is then further cyanomethylated by 10% excess of formaldehyde and hydrocyanic acid in acid solution to EDTN to determine the degree of conversion of adduct to EDDiN under continuous conditions based upon the concentration of hydrocyanic acid in the first stage.

Two test runs were made with the first stage cyanomethylation temperatures maintained at 30°C with a 30 minutes residence time. The final EDTN yields on these tests were:

| Mole Ratio EDA/HCHO/HCN | % EDTN Yield (based on EDA) |
|---|---|
| 1:2.0:0.72 | 35% |
| 1:2.0:2.2 | 50% |

In the second phase of testing of the continuous reaction, EDDiN is cyanomethylated to the tetraacetonitrile in a continuous stirred reactor with an acid mixture of formaldehyde and hydrocyanic acid in 10% excess over stoichiometric.

Charge one mole of EDDiN solution (prepared in the semi-batch procedure of Example 1) in portions, as needed, to a jacketed reservoir, cooled with ice water, leading via a metering pump to the first stage of a cascaded series of insulated one-liter CSTR vessels equipped with a side-arm overflow pipe to the next vessel.

Charge as needed mixture of 713 g of water, 54.8 g of sulphuric acid (96%), 144 g of 50% aqueous formaldehyde and 64.8 g of 100% hydrocyanic acid to a reservoir, fitted with an ice water cooled Friedrichs condenser, and connected via an adjustable metering pump to the same CSTR cascade.

Pump the EDDiN solution and the acidic formaldehyde/hydrocyanic acid mixture to the first-stage of the cascade. Residence times stoichiometry are controlled and determined by the total pumping rate and pumping rate-ratio respectively. Collect the ETTN slurry from the last-stage overflow in ice, over uniform increments of time. Each increment collection is filtered, wqashed and dried and segregated.

In the two and three stage cascades with residence times of 3 and 1 hour respectively, overall yields of 96 and 94% based on EDDiN are obtained.

Example 3: (Continuous hydrolysis EDTN to EDTA·Na$_4$)

The continuous operation of the EDTN hydrolysis to form the EDTA·Na$_4$ stock solution is carried out in a manner similar to the hydrolysis step of Example 1 with the exception that the EDTN/NaOH slurry is pumped into one or more CSTR stages. The overflow of the last CSTR is caught in ice to quench the reaction for incremental yield data.

9

Example 4: (Pilot plant)

(1) EDA/HCHO premix: Charge HCHO (50%) 310.7 kg (269.9 l) to a premixer. Add EDA 155.4 kg (170.7 l) to the premixer over a 3.67 hour period. Reactor temperature is monitored and the feed is adjusted to maintain the temperature at about 30°C.

Transfer the premixer contents 466.1 kg (442.1 l) of EDA/HCHO adduct to a first holding tank.

(2) First-step cyanomethylation (EDDiN Formation): Charge 81.5 kg (1218.9 l) of hydrocyanic acid to weight tank.

Charge 271.5 kg (257.4 l) of the EDA/HCHO adduct to a (stainless steel) reaction vessel from the first holding tank.

Pump hydrocyanic acid from the weight tank to the first reaction vessel over a 1.33 hour period. The reactor temperature is monitored and the feed is adjusted to maintain temperature at 30°C.

After completion of the hydrocyanic acid addition, hold the reaction mass for 0.50 hours at 30°C.

(3) Second-step cyanomethylation (EDTN formation): Charge 666.5 kg (653.0 l) of filtrate at 30°C from the latter EDTN separation step to a second glass-lined reaction vessel.

Meter 43.1 kg (2.3 l) of sulphuric acid to the second reaction vessel, and charge 96.0 kg (83.3 l) formaldehyde to the second vessel together with 43.4 kg (64.7 l) hydrocyanic acid.

Adjust the pH-value of the reactants in the vessel as required to a pH-value of 1.0 by additional sulphuric acid. The temperature is maintained at 30°C.

Add 359.3 kg EDDiN solution from the first reaction vessel to the second reactor over a 1.0 hour period. The temperature is allowed to rise adiabatically to 60—65°C over the course of the addition period. The pH-value will drop to 0.5—0.8 during the course of the addition.

Allow the 1860.2 kg (1770.0 l) contents of the second reactor of EDTN slurry to settle in the reactor after the agitator has been turned "off".

(4) EDTN isolation: After the slurry has settled, withdraw 666.5 kg (653.0 l) of the supernatant solution, and chill to 30°C and set aside in a hold tank. No attempt is made to remove any EDTN crystals which may form.

Add 662.4 l of chilled water to the remaining contents of the reactor restart the agitator and pump the resulting cool slurry to a holding tank containing 87.1 kg of 50% caustic soda and 90.7 kg of water which is at a temperature of 20°C. After all the slurry has been added, the pH-value is in the range of 9.0—10.0 and the temperature is less than 50°C. At this point use one of the two following procedures:

1) Centrifuge and wash the alkaline slurry with water until free of sulfate ion (BaCl$_2$ to wash water gives no cloud). The wet cake will contain from 5 to 10% water. The alkaline mother liquor and wash water are stored in a hold tank for further use. Transfer the washed EDTN wet cake (90%—95% solids) 261.3 kg to a slurry tank where 984.2 l water is charged to give a 26% EDTN slurry (stock solution).

2) Pump the partially cooled slurry directly to the saponification vessel to which is added 43.5 kg of caustic soda (dry basis) in addition to the regular caustic charge as well as any alkaline mother liquor and wash water from previous runs in which EDTN was isolated and washed. If these solutions are used, no water should be added to the normal saponification charge.

Charge 544.3 kg of sodium hydroxide to a hydrolysis reactor and add 257.4 l water. Heat the contents of the reactor to about 100°C. Add 2.7 kg of EDTN slurry from the slurry tank, to the hydrolysis reactor over a 4 hour period.

Hold the reactor at distillation temperature for an additional hour to boil off ammonia. (The aqueous ammonia vapors are vented to an ammonia scrubber).

Slowly charge formaldehyde to the hydrolysis reactor. The concentration of hydrocyanic acid is monitored and the formaldehyde feed is stopped when the acid concentration in the reactor is less than 10 ppm.

Cool the reactor contents from 100° to 80°C and slowly charge hydrogen peroxide while the APHA (American Public Health Association) colour is monitored. The hydrogen peroxide feed is discontinued when the colour is less than 375.

The product concentration is determined and demineralized water is added to give a solution concentration of 30% as EDTA acid.

The EDTA·Na$_4$ bulk solution (30% as EDTA) is the product. (Yield based on EDA:≥98%).

**Claims**

1. A process for the production of pure ethylenediamine tetraacetic acid from ethylenediamine, formaldehyde and hydrocyanic acid characterized in that it comprises the steps of

(a) admixing 2 moles of formaldehyde with 1 mole of ethylenediamine to form an adduct thereof,

(b) performing a first stage cyanomethylation of said adduct by reacting it with 2 moles of hydrocyanic acid at a temperature below about 30°C to form ethylenediamine diacetonitrile,

(c) adding at least 2 additional moles each of hydrocyanic acid and formaldehyde to said ethylenediamine diacetonitrile in a second cyanomethylation step in the presence of sufficient acid to maintain the pH-value of the reaction mixture below about 1.0 and temperatures below about 70°C to complete the second state of cyanomethylation and to form ethylenediamine tetraacetonitrile, and

10

**0 085 277**

(d) saponifying the ethylenediamine tetraacetonitrile with a strong caustic solution to form the tetrasodium salt solution of ethylenediamine tetraacetonitrile acid.

2. The process according to claim 1 wherein the hydrocyanic acid in said second cyanomethylation step is added to a 10 mole % excess for each mole of ethylenediamine diacetonitrile used.

3. The process according to claim 1, wherein the admixing step for forming said adduct includes sufficient cooling of the reaction mixture below said 30°C temperature to remove the exotherm.

4. The process according to claim 1, wherein during the initial first stage cyanomethylation, the pH-value of the reaction mixture is maintained at about 8—10 by the rate of addition of the hydrocyanic acid to the adduct.

5. The process according to claim 1, wherein during the reaction of the two moles of hydrocyanic acid with the adduct to form the ethylenediamine diacetonitrile, the reaction mixture is cooled to remove the heat of reaction while maintaining said reaction mixture below said upper temperature limit of about 30°C.

6. The process according to claim 1, wherein during said second stage cyanomethylation, the resulting solution of ethylenediamine diacetonitrile from the first stage cyanomethylation is added to a mixture comprising sulphuric acid, hydrocyanic acid and formaldehyde at a rate so that the pH-value of the reaction mixture is kept below about 1.0.

7. The process according to claim 1, wherein the second stage cyanomethylation reaction is constantly cooled to temperatures below said 70°C limit by the addition of sufficient amounts of cooled mother liquor to the second stage cyanomethylation reaction mixture to moderate the temperature rise caused by the second stage exotherm.

8. The process according to claim 7, wherein the coolant mother liquor is introduced at a temperature of about 20 to 30°C to the acid mixture.

9. The process according to claim 6, wherein the rate of addition of the ethylenediamine diacetonitrile solution to the reaction mixture is controlled in response to the continuous sensing of the pH-value of the second stage cyanomethylation reaction mixture.

10. The process according to claim 1, wherein the reactions are performed in a semi-batch sequence wherein the rates of addition of components during the adduct formation and the first and second stage cyanomethylation steps is carried out to maintain the reaction temperature and/or pH-value within the prescribed limits for each step.

11. The process according to claim 1, wherein the process comprises the continuous addition of raw materials to a series of reaction vessels where each respective step is sequentially performed and the resultant intermediate materials from each step are introduced into the successive vessel with the respective raw materials for said step together with any materials recycled for completion of reaction or cooling.

12. The process according to claim 1, wherein the insoluble ethylenediamine tetraacetonitrile, is separated from the mother liquor, washed and reslurried in water.

13. The process according to claim 1, wherein the ethylenediamine tetraacetonitrile containing slurry is separated from the mother liquor and washed and is then saponified with said caustic solution to form a solution containing tetrasodium ethylenediamine tetraacetic acid.

14. The process according to claim 1, wherein the entire ethylenediamine tetraacetonitrile slurry is neutralized and saponified with said caustic solution to form a solution containing tetrasodium ethylenediamine tetraacetic acid.

15. The process according to claim 1, wherein the ethylenediamine tetraacetonitrile is saponified with a strong caustic solution containing nitrile reaction mother liquor which has been neutrialized with a strong caustic solution.

16. The process according to claims 13, 14 and 15, wherein said solutions of tetrasodium ethylenediamine tetraacetic acid are neutralized and adjusted to a pH-value of 2 to 3 with mineral acid to precipitate crystals of ethylenediamine tetraacetic acid, separating said crystals from the resulting slurry, and then washing said crystals with water until free from salts and mineral acid.

17. The process according to claim 1, wherein in step (c) a hydrocyanic acid and formaldehyde are added at a pH-value of less than 1.0 under adiabatic conditions at a temperature below the reflux temperature of hydrocyanic acid.

18. A process for the production of pure ethylenediamine tetraacetonitrile from ethylenediamine, formaldehyde and hydrocyanic acid characterized in that it comprises the steps of

(a) admixing about 2 moles of formaldehyde with 1 mole of ethylenediamine to form an adduct thereof,

(b) performing a first stage cyanomethylation of said adduct by reacting it with 2 moles of hydrocyanic acid at temperatures below about 30°C to form ethylenediamine diacetonitrile,

(c) adding at least 2 additional moles each of hydrocyanic acid and formaldehyde to the ethylenediamine diacetonitrile in a second cyanomethylation step in the presence of sufficient acid to maintain the pH-value of the reaction mixture below about 1.0 and temperatures below about 70°C to complete the second stage of cyanomethylation and to form ethylenediamine tetraacetonitrile and

(c 1) collecting and recovering the slurry of ethylenediamine tetraacetonitrile and the mother liquor.

# 0 085 277

**Patentansprüche**

1. Verfahren zur Herstellung reiner Aethylendiamintetraessigsäure aus Aethylendiamin, Formaldehyd und Blausäure, dadurch gekennzeichnet, dass man

(a) 2 Mol Formaldehyd mit 1 Mol Aethylendiamin zu einem Addukt umsetzt,

(b) in einem 1. Schritt das Addukt mit 2 Mol Blausäure bei einer Temperatur unterhalb von etwa 30°C zu Aethylendiamindiacetonitril cyanmethyliert,

(c) in einem 2. Schritt das Diacetonitril mit je weiteren 2 Mol Blausäure und Formaldehyd cyanmethyliert, wobei man durch Säurezusatz einen pH-Wert des Reaktionsgemisches innerhalb von etwa 1,0 einstellt und das Diacetonitril bei Temperatur unterhalb von etwa 70°C zu Aethylendiamintetraacetonitril vollständig cyanmethyliert und

(d) das Aethylendiamintetraacetonitril mit einer starken ätzalkalischen Lösung zu einer Lösung des Tetranatriumsalzes der Aethylendiamintetraessigsäure verseift.

2. Verfahren nach Anspruch 1, dadurch gekennzeichnet, dass man in der 2. Stufe der Cyanmethylierung Blausäure in einem Ueberschuss von 10 Mol%,bezogen auf 1 Mol Aethylendiamindiacetonitril, einsetzt.

3. Verfahren nach Anspruch 1, dadurch gekennzeichnet, dass man bei der Umsetzung von Formaldehyd und Aethylendiamin das Reaktionsgemisch so kühlt, dass sich die Temperatur des Reaktionsgemisches durch die exotherme Reaktion unterhalb von 30°C einstellt.

4. Verfahren nach Anspruch 1 dadurch gekennzeichnet, dass man beim 2. Schritt der Cyanmethylierung die Zulaufgeschwindigkeit der Blausäure so steuert, dass der pH-Wert des Reaktionsgemisches etwa 8 bis 10 beträgt.

5. Verfahren nach Anspruch 1, dadurch gekennzeichnet, dass man bei der Umsetzung des Adduktes mit 2 Mol Blausäure zu Aethylendiamindiacetonitril das Reaktionsgemisch so kühlt, dass sich die Temperatur des Reaktionsgemisches durch die exotherme Reaktion unterhalb von etwa 30°C einstellt.

6. Verfahren nach Anspruch 1, dadurch gekennzeichnet, dass man beim. 2. Schritt der Cyanmethylierung die Zulaufgeschwindigkeit des Aethylendiamindiacetonitrils in die Mischung von Schwefelsäure, Blausäure und Formaldehyd so steuert, dass der pH-Wert des Reaktionsgemisches unterhalb von etwa 1,0 gehalten wird.

7. Verfahren nach Anspruch 1, dadurch gekennzeichnet, dass man beim 2. Schritt der Cyanmethylierung dafür sorgt, dass durch Zusatz von gekühlter Mutterlauge zum Reaktionsgemisch sich die Temperatur des Reaktionsgemisches durch die exotherme Reaktion nicht über 70°C erhöht.

8. Verfahren nach Anspruch 7, dadurch gekennzeichnet, dass man die zu Kühlzwecken eingesetzte Mutterlauge zum Säuregemisch bei etwa 20 bis 30°C gibt.

9. Verfahren nach Anspruch 6, dadurch gekennzeichnet, dass man die Zulaufgeschwindigkeit des Aethylendiamindiacetonitrils zum Reaktionsgemisch durch die kontinuierliche Messung des pH-Wertes des Reaktionsgemisches beim 2. Schritt der Cyanmethylierung steuert.

10. Verfahren nach Anspruch 1, dadurch gekennzeichnet, dass die Reaktionsführung halb ansatzweise erfolgt, wobei man dei Zulaufgeschwindigkeit der Komponenten bei der Adduktbildung beim 1. und 2. Schritt der Cyanmethylierung so steuert, dass die Reaktionstemperatur und/oder der pH-Wert innerhalb der vorgeschriebenen Grenzen für jede Verfahrensstufe eingehalten wird.

11. Verfahren nach Anspruch 1, dadurch gekennzeichnet, dass die Zugabe der Ausgangsmaterialien kontinuierlich erfolgt, wobei eine Reihe von Reaktionsgefässen eingesetzt wird, worin jede der entsprechenden Verfahrensschritte aufeinanderfolgend durchgeführt wird und wobei das Zwischenprodukt der jeweiligen Verfahrensschritte in das nachfolgende Reaktionsgefäss zusammen mit dem für diesen nächsten Verfahrensschritt benötigten Ausgangsmaterial und allfälligen wiedergewonnenen Materialien zur Vervollständigung der Reaktion oder zu Kühlzwecken eingeführt wird.

12. Verfahren nach Anspruch 1, dadurch gekennzeichnet, dass man das unlösliche Aethylendiamintetraacetonitril von der Mutterlauge abtrennt, nachwäscht und in Wasser widerum anschlämmt.

13. Verfahren nach Anspruch 1, dadurch gekennzeichnet, dass man die Aethylendiamintetraacetonitril enthaltende Anschlämmung von der Mutterlauge abtrennt, nachwäscht und mit der ätzalkalischen Lösung zu einer Lösung des Tetranatriumsalzes der Aethylendiamintetraessigsäure verseift.

14. Verfahren nach Anspruch 1, dadurch gekennzeichnet, dass man die gesamte Anschlämmung von Aethylendiamintetraacetonitril mit der ätzalkalischen Lösung zu einer Lösung des Tetranatriumsalzes der Aetylendiamintetraessigsäure verseift.

15. Verfahren nach Anspruch 1, dadurch gekennzeichnet, dass man Aethylendiamintetraacetonitril mit einer starken ätzalkalischen Lösung verseift, welche die Nitril aufweisende Mutterlauge enthält und mit einer starken ätzalkalischen Lösung neutralisiert worden ist.

16. Verfahren nach einem der Ansprüche 13, 14 oder 15, dadurch gekennzeichnet, dass man die wässrigen Lösungen vom Tetranatriumsalz der Aethylendiamintetraessigsäure mit einer Mineralsäure netralisiert und auf einen pH-Wert von 2 bis 3 einstellt, um Kristalle der Aethylendiamintetraessigsäure zu fällen, diese Kristalle von der erhaltenen Anschlämmung trennt und mit Wasser salzfrei und säurefrei nachwäscht.

17. Verfahren nach Anspruch 1, dadurch gekennzeichnet, dass man beim Verfahrensschritt (c) Blausäure und Formaldehyd bei einem pH-Wert von weniger als 1,0 unter adiabatischen Bedingungen und bei Temperaturen unterhalb der Rückflusstemperatur der Bläusäure zusetzt.

18. Verfahren zur Herstellung von reinem Aethylendiamintetraacetonitril aus Aethylendiamin, Formaldehyd und Blausäure, dadurch gekennzeichnet, dass man

(a) etwa 2 Mol Formaldehyd mit 1 Mol Aethylendiamin zu einem Addukt umsetzt,

(b) in einem 1. Schritt das Addukt mit 2 Mol Blausäure bei einer Temperatur unterhalb von etwa 30°C zum Aethylendiamindiacetonitril cyanmethyliert,

(c) in einem 2. Schritt das Diacetonitril mit je weiteren 2 Mol Blausäure und Formaldehyd cyanmethyliert, wobei man durch Säurezusatz einen pH-Wert des Reaktionsgemisches unterhalb von etwa 1,0 einstellt und das Diacetonitril bei Temperaturen unterhalb von etwa 70°C zum Aethylendiamintetraacetonitril vollständig cyanmethyliert und

(c$_1$) die Anschlämmung von Aethylendiamintetraacetonitril und die Mutterlauge auffängt und wieder verwendet.

## Revendications

1. Procédé de préparation d'acide éthylènediamine-tétracétique pur à partir d'éthylènediamine, de formaldéhyde et d'acide cyanhydrique, caractérisé par le fait qu'il comprend les étapes consistant à

(a) mélanger 2 moles de formaldéhyde avec 1 mole d'éthylènediamine pour constituer un produit d'addition de ceux-ci,

(b) effectuer un premier stade de cyanométhylation dudit produit d'addition en le faisant réagir avec 2 moles d'acide cyanhydrique à une température inférieure à 30° C pour former de l'éthylènediamine-diacétonitrile,

(c) ajouter au moins 2 moles supplémentaires de chaque composé acide cyanhydrique et formaldéhyde audit éthylènediamine-diacétonitrile dans une seconde étape de cyanométhylation en présence d'une quantité suffisante d'acide pour maintenir le pH du mélange réactionnel à une valeur inférieure à environ 1,0 et des températures inférieures à environ 70° C pour achever le second stade de cyanométhylation et pour former de l'éthylènediamine-tétracétonitrile, et

(d) saponifier l'éthylènediamine-tétracétonitrile avec une solution fortement alcaline pour former la solution de sel tétrasodique de l'acide éthylènediamine-tétracétique.

2. Procédé selon la revendication 1, dans lequel on ajoute dans ladite deuxième étape de cyanométhylation l'acide cyanhydrique en un excès de 10% en mole par mole d'éthylènediamine-diacétonitrile utilisé.

3. Procédé selon la revendication 1, dans lequel l'étape de mélange pour former ledit produit d'addition comprend un refroidissement suffisant du mélange réactionnel au-dessous de ladite température de 30° C pour éliminer la chaleur dégagée par la réaction.

4. Procédé selon la revendication 1, dans lequel le pH du mélange réactionnel est maintenu pendant la cyanométhylation initiale de la première étape à une valeur d'environ 8—10 par la cadence d'addition de l'acide cyanhydrique au produit d'addition.

5. Procédé selon la revendication 1, dans lequel, pendant la réaction des deux moles d'acide cyanhydrique avec le produit d'addition pour former l'éthylènediamine-diacétonitrile, le mélange réactionnel est refroidi pour éliminer la chaleur de réaction tout en maintenant ledit mélange réactionnel au-dessous de ladite limite supérieure de température d'environ 30° C.

6. Procédé selon la revendication 1, dans lequel, au cours de ladite cyanométhylation du deuxième stade, la solution d'éthylènediamine-diacétonitrile résultant du premier stade de cyanométhylation est ajoutée à un mélange comprenant de l'acide sulfurique, de l'acide cyanhydrique et du formaldéhyde à une cadence telle que le pH du mélange réactionnel est maintenu à une valeur inférieure à environ 1,0.

7. Procédé selon la revendication 1, dans lequel la réaction de cyanométhylation du deuxième stade est continuellement refroidie à des températures inférieures à ladite limite de 70° C par l'addition au mélange réactionnel de cyanométhylation du deuxième stade de quantités suffisantes de liqueur-mère refroidie pour modérer l'élévation de température provoquée par le dégagement de chaleur du deuxième stade.

8. Procédé selon la revendication 7, dans lequel la liqueur-mère refroidissante est introduite dans le mélange d'acides à une température de environ 20 à 30° C.

9. Procédé selon la revendication 6, dans lequel la cadence d'addition au mélange réactionnel de la solution d'éthylènediamine-diacétonitrile est commandée en répose à la mesure continuelle de la valeur du pH du mélange réactionnel de cyanométhylation du deuxième stade.

10. Procédé selon la revendication 1, dans lequel les réactions sent mises en oeuvre dans une séquence semi-continue dans laquelle les cadences d'addition des composants pendant la formation du produit d'addition et les étapes de cyanométhylation du premier et du deuxième stade sont effectuées pour maintenir la température et/ou le pH de la réaction à l'intérieur de limites prescrites pour chaque étape.

11. Procédé selon la revendication 1, dans lequel le procédé comprend l'addition en continu de matériaux de base dans une série de récipients laboratoires où chaque étape respective est effectuée séquentiellement et les produits intermédiaires résultant de chaque étape sont introduits dans le récipient successif avec les matériaux de base respectifs pour ladite étape conjointement à tout matériau recyclé pour l'achèvement de la réaction ou le refroidissement.

12. Procédé selon la revendication 1, dans lequel l'éthylènediamine-tétracétonitrile insoluble est séparé de la liqueur-mère, lavé et remis en suspension dans de l'eau.

13. Procédé selon la revendication 1, dans lequel la suspension contenant l'éthylènediamine-tétracéto-nitrile est séparée de la liqueur-mère et lavée et elle est ensuite saponifiée avec ladite solution caustique pour former une solution contenant de l'acide éthylènediamine-tétracétique tétrasodique.

14. Procédé selon la revendication 1, dans lequel la totalité de la suspension d'éthylènediamine-tétracétonitrile est neutralisée et saponifiée avec ladite solution caustique pour former une solution contenant de l'acide éthylènediamine-tétracétique tétrasodique.

15. Procédé selon la revendication 1, dans lequel l'éthylènediamine-tétracétonitrile est saponifié avec une liqueur-mère de réaction du nitrile contenant du la solution fortement alcaline, qui a été neutralisée avec une solution fortement alcaline.

16. Procédé selon les revendications 13, 14 et 15, dans lequel lesdites solutions d'acide éthylènediamine-tétracétique tétrasodique sont neutralisées et leur pH est ajusté à une valeur de 2 à 3 avec un acide minéral pour précipiter des cristaux d'acide éthylènediamine-tétracétique, on sépare lesdits cristaux de la suspension résultante, et on lave ensuite lesdits cristaux avec de l'eau jusqu'a ce qu'ils soient exempts de sels et d'acide minéral.

17. Procédé selon la revendication 1, dans lequel on ajoute a l'étape (c) de l'acide cyanhydrique et du formaldéhyde à un pH ayant une valeur inférieure à 1,0 dans des conditions adiabatiques à une température inférieure à la température de reflux de l'acide cyanhydrique.

18. Procédé de préparation d'éthylènediamine-tétracétonitrile pur à partir d'éthylènediamine, de formaldéhyde et d'acide cyanhydrique, caractérisé par le fait qu'il comprend les étapes qui consistent à

(a) mélanger environ 2 moles de formaldéhyde avec 1 mole d'éthylènediamine pour former un produit d'addition de ceux-ci,

(b) effectuer un premier stade de cyanométhylation dudit produit d'addition en le faisant réagir avec 2 moles d'acide cyanhydrique à des températures inférieures à environ 30° C pour former de l'éthylènediamine-diacétonitrile.

(c) ajouter au moins 2 moles supplémentaires de chacun des composés acide cyanhydrique et form-aldèhyde à l'éthylènediamine-diacétonitrile dans une deuxième étape de cyanométhylation en présence d'une quantité suffisante d'acide pour maintenir le pH du mélange réactionnel à une valeur inférieure à environ 1,0 et des températures inférieures à environ 70° C pour achever le deuxième stade de cyanométhylation et pour former de l'éthylènediamine-tétracétonitrile et

$(c_1)$ recueillir et récupérur la suspension d'éthylènediamine-tétracétonitrile et la liqueur-mère.